# EUROPEAN PATENT SPECIFICATION

(11) **EP 1 223 950 B1**
(45) Date of publication and mention of the grant of the patent: **04.01.2006**
(21) Application number: 00975399.7
(22) Date of filing: 26.10.2000
(51) Int. Cl.: A61K 31/713, C12N 15/11, C12N 9/00, C07H 21/00, A61P 27/02

(54) **RIBOZYME THERAPY FOR THE TREATMENT OF PROLIFERATIVE EYE DISEASES**
RIBOZYM-THERAPIE ZUR BEHANDLUNG VON PROLIFERATIVEN AUGENKRANHEITEN
THERAPIE RIBOZYMIQUE DESTINEE AU TRAITEMENT DE MALADIES PROLIFERATIVESS DES YEUX

(30) Priority: 26.10.1999 US 161532 P
(43) Date of publication of application: 24.07.2002
(73) Proprietor: IMMUSOL, INC., San Diego, CA 92121 (US)
(72) Inventor: ROBBINS, Joan, M., San Diego, CA 92131 (US); TRITZ, Richard, San Diego, CA 92121 (US)
(74) Representative: Walton, Seán Malcolm
(86) International application number: PCT/US2000/029500
(87) International publication number: WO 2001/030362

(56) References cited:
- WO-A-00/32765
- WO-A-91/15580
- WO-A-97/10334
- WO-A-99/50403
- FRIMERMAN AARON ET AL: "Chimeric DNA-RNA hammerhead ribozyme to proliferating cell nuclear antigen reduces stent-induced stenosis in a porcine coronary model." CIRCULATION, vol. 99, no. 5, 9 February 1999 (1999-02-09), pages 697-703, XP002246003 ISSN: 0009-7322
- OZAKI, H. ET AL.: "INTRAVITREAL SUSTAINED RELEASE OF VEGF CAUSES RETINAL NEOVASCULARIZATION IN RABBITS AND BREAKDOWN OF THE BLOOD-RETINAL BARRIER IN RABBITS AND PRIMATES" EXPERIMENTAL EYE RESEARCH, vol. 64, 1997, pages 505-517, XP000909325 ISSN: 0014-4835

## Description

### TECHNICAL FIELD

The present invention relates generally to therapeutics, and more specifically, to compositions which may be utilized in the treatment and/or prevention of proliferative eye diseases, such as proliferative vitreo retinopathy and proliferatine diabetic retinopathy

### BACKGROUND OF THE INVENTION

Proliferative diseases of the eye such as proliferative diabetic retinopathy (PDR) affect as many as 700,000 in the U.S., with more than 65,000 new cases diagnosed each year. Annually, as many as 25,000 people go blind from the disorder, making it a leading cause of blindness among working-age Americans. The cost savings from successful intervention could be as high as 100 million dollars per year. The disease does not stem from a single retinal change. Rather, it may be triggered by a combination of biochemical, metabolic, and hematological abnormalities.

There are no early symptoms. There is no pain, no blurred vision, and no ocular inflammation. In fact, many people do not develop any visual impairment until the disease has advanced well into its proliferative stage. At this point, the vision that has been lost cannot be restored. Laser surgery, also called photocoagulation, is the only current therapy to treat PDR. At present there is no cure for this disease.

Thus, a need exists for an effective therapy to treat proliferative diseases (*e.g.*, PDR). The present invention satisfies this need and further provides other related advantages as well.

### SUMMARY OF THE INVENTION.

The present invention provides use of a chimeric DNA/RNA hammerhead ribozyme in the manufacture of a medicament for treatment of a proliferative disease of the eye, wherein the ribozyme is directed to the sequence of any of SEQ ID NOS: 1-261, 271-280, wherein these sequences are within the PCNA gene.

The medicament may inhibit retinal detachment.

A preferred ribozyme for use in the present invention is directed to SEQ ID NO: 273.

The proliferative disease of the eye may be proliferative vitreoretinopathy (PVR) or proliferative diabetic retinopathy (PDR) .

These and other aspects of the present invention will become evident upon reference to the following detailed description and attached drawings.

### BRIEF DESCRIPTION OF THE DRAWINGS

Figure 1 is a schematic illustration of which shows the general structure of a chimeric DNA/RNA ribozyme (SEQ ID NOs: 281 and 282).
Figure 2 is a photograph of a gel which shows the stability of chimeric ribozymes PN30003, 30004, and 30005 in human vascular smooth muscle cell lysate.
Figure 3 is a photograph of a gel which shows the stability of chimeric ribozymes PN30003 and 30005 in serum.
Figure 4 is a schematic illustration of a propanediol linker.
Figure 5 is a photograph of control eyes and eyes treated with chimeric PCNA targeted ribozyme from a rabbit dispase model of proliferative vitreoretinopathy.
Figure 6 is a chart of histopathologic scoring of chimeric PCNA targeted ribozyme versus control treated rabbit eyes. Scoring is based on a modified Fastenberg scale (Table 22) and performed blindly by two trained observers. P is less than 0.0008 by T test.

### DETAILED DESCRIPTION OF THE INVENTION

### DEFINITIONS

Prior to setting forth the invention, it may be helpful to an understanding thereof to first set forth definitions of certain terms that will be used hereinafter.

"Ribozyme" refers to a nucleic acid molecule which is capable of cleaving a specific nucleic acid sequence. Ribozymes may be composed of RNA, DNA, nucleic acid analogues (*e.g.*, phosphorothioates), or any combination of these (*e.g*., DNA/RNA chimerics). Within particularly preferred embodiments, a ribozyme should be understood to refer to RNA molecules that contain anti-sense sequences for specific recognition, and an RNA-cleaving enzymatic activity.

"Ribozyme gene" refers to a nucleic acid molecule (*e.g.*, DNA) consisting of the ribozyme sequence which, when transcribed into RNA, will yield the ribozyme.

"Vector" refers to an assembly which is capable of expressing a ribozyme of interest. The vector may be composed of either deoxyribonucleic acids ("DNA") or ribonucleic acids ("RNA"). Optionally, the vector may include a polyadenylation sequence, one or more restriction sites, as well as one or more selectable markers. such as neomycin phosphotransferase, hygromycin phosphotransferase or puromycin-N-acetyl-transferase. Additionally, depending on the host cell chosen and the vector employed, other genetic elements such as an origin of replication, additional nucleic acid restriction sites, enhancers, sequences conferring inducibility of transcription, and selectable markers, inay also be incorporated into the vectors described herein.

"Nucleic acid" or "nucleic acid' molecule" refers to any of deoxyribonucleic acid (DNA), ribonucleic acid (RNA), oligonucleotides, fragments generated by the polymerase chain reaction (PCR), and fragments generated by any of ligation, scission, endonuclease action, and exonuclease action. Nucleic acids can be composed of monomers that are naturally-occurring nucleotides (such as deoxyribonucleotides and ribonucleotides), or analogs of naturally-occurring nucleotides (*e*.*g*., α-enantiomeric forms of naturally-occurring nucleotides), or a combination of both. Modified nucleotides can have modifications in sugar moieties and/or in pyrimidine or purine base moieties. Sugar modifications include, for example, replacement of one or more hydroxyl groups with halogens, alkyl groups, amines, and azido groups, or sugars can be functionalized as ethers or esters. Moreover, the entire sugar moiety can be replaced with sterically and electronically similar structures, such as aza-sugars and carbocyclic sugar analogs. Examples of modifications in a base moiety include alkylated purines and pyrimidines, acylated purines or pyrimidines, or other well-known heterocyclic substitutes. Nucleic acid monomers can be linked by phosphodiester bonds or analogs of such linkages. Analogs of phosphodiester linkages include phosphorothioate, phosphorodithioate, phosphoroselenoate, phosphorodiselenoate, phosphoroanilothioate, phosphoranilidate, phosphoramidate, and the like. The term "nucleic acid" also includes so-called "peptide nucleic acids," which comprise naturally-occurring or modified nucleic acid bases attached to a polyamide backbone. Nucleic acids can be either single stranded or double stranded.

"Isolated nucleic acid molecule" is a nucleic acid molecule that is not integrated in the genomic DNA of an organism. For example, a DNA molecule that encodes a gene that has been separated from the genomic DNA of a eukaryotic cell is an isolated DNA molecule. Another example of an isolated nucleic acid molecule is a chemically-synthesized nucleic acid molecule that is not integrated in the genome of an organism.

"Promoter" is a nucleotide sequence that directs the transcription of a structural gene. Typically, a promoter is located in the 5' region of a gene, proximal to the transcriptional start site of a structural gene. If a promoter is an inducible promoter, then the rate of transcription increases in response to an inducing agent. In contrast, the rate of transcription is not regulated by an inducing agent if the promoter is a constitutive promoter.

As noted above, proliferative diseases such as PDR, affect many individuals and represent a major cost to the health care system. As discussed in more detail below, by interfering with cell-cycle control of cells which might otherwise proliferate, proliferative diseases such as PDR or PVR can be effectively treated. This invention accomplishes such by providing use of ribozymes

Cyclins and cell-cycle dependent kinases are directly involved in the signaling and synthesis that results in cells progressing through the cell cycle to replicate. Representative examples of suitable ribozyme targets include PCNA HH ribozyme binding sites (SEQ ID NOS: 3855-4115).

### RIBOZYMES

As noted above, the present invention employs chimeric DNA/RNA hammerhead ribozymes having the ability to cleave or otherwise inhibit PCNA Several different types of ribozymes are available in the art and are discerned here as background, including for example, hammerhead ribozymes (Rossi, J.J. et al., *Pharmac. Ther.* 50:245-254, 1991) (Forster and Symons, *Cell 48*:211-220, 1987; Haseloff and Gerlach, *Nature* 328:596-600, 1988; Walbot and Bruening, *Nature 334*:196, 1988; Haseloff and Geriach, *Nature 334*:585, 1988; Haseloff et al., U.S. Patent No. 5,254,678), hairpin ribozymes (Hampel et al., *Nucl. Acids Res. 18*:299-304, 1990, and U.S. Patent No. 5,254,678), hepatitis delta virus ribozymes (Perrotta and Been, *Biochem. 31:16,* 1992), Group I intron ribozymes (Cech et al., U.S. Patent No. 4,987,071) and RNase P ribozymes (Takada et al., *Cell 35*:849, 1983); *(see* also, WO 95/29241,. entitled "Ribozymes with Product Ejection by Strand Displacement"; and WO 95/31551, entitled "Novel Enzymatic RNA Molecules."

Cech et al. (U.S. Patent No. 4,987,671, issued January 22, 1991) has disclosed the preparation and use of ribozymes which are based on the properties of the *Tetrahymena* ribosomal RNA self-splicing reaction. These ribozymes require an eight base pair target site and free guanosine (or guanosine derivatives). A temperature optimum of 50°C is reported for the endoribonuclease activity. The fragments that arise from cleavage contain 5'-phosphate and 3'-hydroxyl groups and a free guanosine nucleotide added to the 5'-end of the cleaved RNA.

In contrast to the ribozymes of Cech et al., particularly preferred ribozymes hybridize efficiently to target sequences at physiological temperatures, making them suitable for use *in vivo,* and not merely as research tools (see column 15, lines 18 to 42, of Cech et al., U.S. Patent No. 4,987,071). Thus, particularly preferred ribozymes include hairpin ribozymes (for example, as described by Hampel et al., European Patent Publication No. 0 360 257, published March 26, 1990) and hammerhead ribozymes. Briefly, the sequence requirement for the hairpin ribozyme is any RNA sequence consisting of NNNBN*GUC(N), (Sequence ID Nos. 266-270) (where x is any number from 6 to 10, N*G is the cleavage site, B is any of G, C, or U, and N is any of G, U, C, or A). Additionally, the backbone or common region of the hairpin ribozyme can be designed using the nucleotide sequence of the native hairpin ribozyme (Hampel et al., *Nucl. Acids Res. 18*:299-304, 1990) or it can be modified to include a "tetraloop" structure that increases stability and catalytic activity (see Example 2; see also Yu et al., *Virology 206*:381-386, 1995; Cheong et al., *Nature 346*:680-682, 1990; Anderson et al., *Nucl. Acids Res. 22*:1096-1100**,** 1994).

The sequence requirement at the cleavage site for the hammerhead ribozyme is any RNA sequence consisting of NUH (where N is any of G, U, C, or A and H represents C, U, or A) can be targeted. Accordingly, the same target within the hairpin leader sequence, GUC, is useful for the hammerhead riboryme. The additional nucleotides of the hammerhead ribozyme or hairpin ribozyme is determined by the target flanking nucleotides and the hammerhead consensus sequence *(see* Ruffner et al., *Biochemistry 29*:10695*-*10702, 1990). This information, along with the sequences and disclosure provided herein, enables the production of hairpin ribozymes of this invention.

The ribozymes used in this invention, as well as DNA encoding such ribozymes and other suitable nucleic acid molecules, described in more detail below, can be chemically synthesized using methods well known in the art for the synthesis of nucleic acid molecules (see *e.g.,* Heidenreich et al., *J*. *FASEB 70*(1):90-6, 1993; Sproat, *Curr. Opin. Biorechnol. 4*(1):20-28, 1993). Alternatively, commercial suppliers such as Promega, Madison, Wis., USA, provide a series of protocols suitable for the production of nucleic acid molecules such as ribozymes.

Within one aspect of the present invention, ribozymes are prepared from a DNA molecule or other nucleic acid molecule (which, upon transcription, yields an RNA molecule) operably linked to an RNA polymerase promoter, *e.g*., the promoter for T7 RNA polymerase or SP6 RNA polymerase. Accordingly, also provided by this invention are nucleic acid molecules, *e.g.*, DNA or cDNA, coding for the ribozymes of this invention. When the vector also contains an RNA polymerase promoter operably linked to the DNA molecule, the ribozyme can be produced *in vitro* upon incubation with the RNA polymerase and appropriate nucleotides. In a separate embodiment, the DNA may be inserted into an expression cassette, such as described in Cotten and Bimstiel, *EMBO J. 8*(12):3861-3866, 1989, and in Hempel et al., *Biochemistry 28*:4929-4933, 1989. A more detailed discussion of molecular biology methodology is disclosed in Sambrook et al., *Molecular Cloning: A Laboratory Manual,* Cold Spring Harbor Press, 1989.

During synthesis, the ribozyme can be modified by ligation to a DNA molecule having the ability to stabilize the ribozyme and make it resistant to RNase (Rossi et al., *Pharmac. Ther. 50*:245-254, 1991). The ribozyme can be modified to a phosphothio-analog for use in liposome delivery systems. This modification also renders the ribozyme resistant to endonuclease activity. The ribozyme can be modified to contain propanediol linkages or to incorporate 2'-O-methylated nucleotides.

### VECTORS

Use of ribozymes to treat proliferative skin diseases such as psoriasis or eczema involves introduction of functional ribozyme to the infected cell of interest. This can be accomplished by either synthesizing functional ribozyme *in vitro* prior to delivery, or, by delivery of DNA capable of driving ribozyme synthesis *in vivo*.

More specifically, the ribozyme gene may be constructed within a vector which is suitable for introduction to a host cell (*e.g*., prokaryotic or eukaryotic cells in culture or in the cells of an organism). Appropriate prokaryotic and eukaryotic cells can be transfected with an appropriate transfer vector containing the nucleic acid molecule encoding a ribozyme of this invention.

To produce the ribozymes with a vector *in vivo,* nucleotide sequences coding for ribozymes are preferably placed under the control of a eukaryotic promoter such as pol III (*e.g*., tRNA or VA-1 from adenovirus), CMV, SV40 late, or SV40 early promoters. Within certain embodiments, the promoter may be a tissue or cell-specific promoter. Ribozymes may thus be produced directly from the transfer vector *in vivo*.

A wide variety of vectors may be utilized within the context of the present invention, including for example, plasmids, viruses, retrotransposons and cosmids. Representative examples include adenoviral vectors (*e.g*., WO 94/26914, WO 93/9191; Yei et al., *Gene Therapy 1:*192-200, 1994; Kolls et al., *PNAS 91*(1):215-219, 1994; Kass-Eisler et al., *PNAS 90*(24):11498-502, 1993; Guzman et al., *Circulation 88*(6):2838-48, 1993; Guzrnan et al., *Cir. Res.* 73(6):1202-1207, 1993; Zabner et al., *Cell 75*(2):207-216, 1993; Li et al., *Hum Gene Ther. 4*(4):403-409, 1993; Caillaud et al., *Eur. J. Neurosci. 5*(10):1287-1291, 1993), adeno-associated type 1 ("AAV-1'') or adeno-associated type 2 ("AAV-2") vectors *(see* WO 95/13365; Flotte et a1., *PNAS 90*(22):10613-10617, 1993); hepatitis delta vectors, live, attenuated delta viruses and herpes viral vectors (*e.g*., U.S. Patent No. 5,288,641), as well as vectors which are disclosed within U.S. Patent No. 5,166,320. Other representative vectors include retroviral vectors (*e.g*., EP 0 415 731; WO 90/07936; WO 91/02805; WO 94/03622; WO 93/25698; WO 93/25234; U.S. Patent No. 5,219,740; WO 93/11230; WO 93/10218). General methods of using such vectors in gene therapy are well known in the art (see, for example, Larrick, J.W. and Burck, K.L., *Gene Therapy: Application of Molecular Biology,* Elsevier Science Publishing Co., Inc., New York, New York, 1991 and Kreigler, M., *Gene Transfer and EXpression: A Laboratory Manual,* W.H. Freeman and Company, New York, 1990).

Vectors may have more than one nucleic acid molecule encoding a ribozyme, each molecule under the control of a separate eukaryotic promoter (or, an Internal Ribosome Entry Site or "IRES") or alternatively, under the control of single eukaryotic promoter. Representative examples of other nucleic acid molecules which may be delivered by vectors include therapeutic molecules such as interferon (*e.g*., alpha, beta or gamma), as well as a wide variety of other cytokines or growth factors, and facilitators which assist or aid ribozymes in cleaving a target sequence by unwinding or otherwise limiting secondary folding which might otherwise inhibit the ribozyme These vectors provide the advantage of providing multi-functional therapy against Psoriasis, preferably with the various therapies working together in synergy.

Host prokaryotic and eukaryotic cells stably harboring the vectors described above also are provided by this invention. Suitable host cells include bacterial cells, rat cells, mouse cells, and human cells.

### DELIVERY

Within certain aspects of the invention, ribozyme molecules, or nucleic acid molecules which encode the ribozyme, may be introduced into a host cell or administered to a patient utilizing a vehicle, or by various physical methods. Representative examples of such methods include transformation using calcium phosphate precipitation (Dubensky et al., *PNAS 81*:7529-7533, 1984), direct microinjection of such nucleic acid molecules into intact target cells (Acsadi et al., *Nature* 352:815-818, 1991), and electroporation whereby cells suspended in a conducting solution are subjected to an intense electric field in order to transiently polarize the membrane, allowing entry of the nucleic acid molecules. Other procedures include the use of nucleic acid molecules linked to an inactive adenovirus (Cotton et al., *PNAS* 89:6094, 1990), lipofection (Felgner et al., *Proc. Natl*. *Acad Sci*. *USA 84*:7413-7417,1989), microprojectile bombardment (Williams et al., *PNAS 88*:2726-2730, 1991), polycation compounds such as polylysine, receptor specific ligands, liposomes entrapping the nucleic acid molecules, spheroplast fusion whereby *E*. *coli* containing the nucleic acid molecules are stripped of their outer cell walls and fused to animal cells using polyethylene glycol, viral transduction, (Cline et al., *Pharmac. Ther. 29*:69, 1985; and Friedmann et al., *Science 244*:1275, 1989), and DNA ligand (Wu et al, *J. of Biol. Chem. 264*:16985-16987, 1989). In one embodiment, the ribozyme is introduced into the host cell using a liposome.

Within further embodiments of the invention, additional therapeutic molecules (*e.g.*, interferon) or facilitators may be delivered utilizing the methods described herein. Such delivery may be either simultaneous to, or before or after the delivery of a ribozyme or vector expressing ribozymes.

### PHARMACEUTICAL COMPOSITIONS

As noted above, pharmaceutical compositions (or "medicaments") are provided by this invention. These compositions contain any of the above described ribozymes, DNA molecules or vectors, along with a pharmaceutically or physiologically acceptable carrier, excipient, or, diluent. Generally, such carriers should be nontoxic to recipients at the dosages and concentrations employed. Ordinarily, the preparation of such compositions entails combining the therapeutic agent with buffers, antioxidants such as ascorbic acid, low molecular weight (less than about 10 residues) polypeptides, proteins, amino acids, carbohydrates including glucose, sucrose or dextrins, chelating agents such as EDTA, glutathione and other stabilizers and excipients. Neutral buffered saline or saline mixed with nonspecific serum albumin are exemplary appropriate diluents. Particularly preferred carriers include cholesterols such as DOTAP:cholesterol.

Additional ingredients added to the compositions for the express purpose of inhibiting endogenous ribonucleases include reducing agents such as dithiothreitol, detergents such as sodium dodecyl sulfate, and other agents such as vanidyl nucleotides, aurin tricarbocylic acid, hydrogen peroxide and RNA decoys such as tRNA.

Pharmaceutical compositions of the present invention may also be prepared to contain; or express (*e.g*., if a vector), one or more additional therapeutic molecules (*e.g*., interferon) or facilitators.

The pharmaceutical compositions of the present invention may be prepared for administration by a variety of different routes, including for example, topically, intradermally, or intraocullarly. In addition, pharmaceutical compositions of the present invention may be placed within containers, along with packaging material which provides instructions regarding the use of such pharmaceutical compositions. Generally, such instructions will include a tangible expression describing the reagent concentration, as well as within certain embodiments, relative amounts of excipient ingredients or diluents (*e*.*g*., water, saline or PBS) which may be necessary to reconstitute the pharmaceutical composition

Pharmaceutical compositions of the present invention are useful for both diagnostic and therapeutic purposes.

### THERAPEUTIC USES

The treatment of proliferative diseases such as PVR or PDR are enabled by this invention.

Briefly, cells in response to injury or other insult typically follow at least one of two possible pathways: normal wound healing or exaggerated growth. For normal wound healing accelerated growth and maturation of cells occurs in order to allow healing of the wound as soon as possible.

Proliferative diseases such as PVR and PDR are similar in many respects to the process of wound healing. Namely, cells are created and migrate in a relatively short period of time. However, if the proliferation is too rapid or the signals to proliferate continue for too long a period of time, the cells build up to form thickened lesions. This growth is supported by new blood vessels, as well as the infiltration of a variety of lymphocytes which produce a wide variety of growth factors (that further increases the proliferation of the cells). These cells can produce tissue degrading enzymes which cause the destruction of surrounding tissue. In some cases, the thickened lesions contract, distorting the surface of the skin or retina. The result of the exaggerated growth is the clinical symptoms associated with the proliferative disease.

The present invention provides for treatment of proliferative eye diseases such as PVR or PDR by contacting desired cells with an effective amount of ribozyme of this invention. A suitable "therapeutically effective amount" will depend on the nature and extent of diseased tissue being treated, or, if a medical procedure is contemplated in which abnormal proliferation can be expected, prevented. Such "therapeutically effective amounts" can be readily determined by those of skill in the art using well known methodology, and suitable animal models (*e*.*g*. a rat, rabbit, or porcine model), or, based upon clinical trials. As utilized herein, a patient is deemed to be "treated" if the proliferative eye disease is reversed or inhibited in a patient in a quantifiable manner. Further, a therapeutically effective amount or regimen of treatment should result in: (1) decrease in the frequency, severity, or, duration of clinical symptoms (*e.g*., inflammation, thickening of the tissue, contraction, scaling, "burning", or itching); (2) increase of time in the period of remission; (3) a change in pathological symptoms (*e*.*g*., inhibition of keratinocyte, fibroblast, glia, or retinal pigment epithelium proliferation); (4) prevention of retinal detachment; and/or (5) prevention of visual impairment.

When exogenously delivering the ribozyme, the RNA molecule can be embedded within a stable RNA molecule or in another form of protective environment, such as a liposome. Alternatively, the RNA can be embedded within RNase-resistant DNA counterparts. Cellular uptake of the exogenous ribozyme can be enhanced by attaching chemical groups to the DNA ends, such as cholesteryl moieties (Letsinger et al., *P*.*N*.*A*.*S*., *U.S.A.,* 1989).

The target cell can include but is not limited to cells found in the vitreous of the eye, and the retinal and pigment epithelium layers of the eye.

In addition, the ribozyme may be readily incorporated into a biodegradable polymer, sphere, pleuroinc gel, or the like to aid incorporation into cells.

The ribozyme can be administered in any manner sufficient to achieve the above therapeutic results. Direct intraocular injection of ribozymes in a suitable pharmaceutical vehicle can be used for the management of individual lesions.

The ribozyme may be formulated along with a liquid (*e.g*., DOTAP:cholesterol). In addition, the ribozyme may be formulated with ribonuclease inhibitors, including, but not limited to, reducing agent (*e.g*., dithiothreitol), detergent (*e.g*., sodium dodecyl sulfate), vanidyl nucleotides, aurin tricarboxcylic acid, .hydrogen peroxide, and RNA decoys (*e.g*., tRNAs).

The following examples are offered by way of illustration, and not by way of limitation.

### EXAMPLES

### EXAMPLE 1

### CRITERIA FOR RIBOZYME SITE SELECTION

Selection of Cleavage Sites for Hammerhead Ribozymes Hammerhead ribozymes suitable for use within the present invention preferably recognize the sequence NUH, wherein N is any of G, U, C, or A and H is C, U, or A. The hairpin recognition sites GUC are a subset of the hammerhead recognition sites NUH. Therefore, all hairpin sites are by definition also hammerhead sites, although the converse is not true. Representative GUC hairpin/hammerhead ribozyme recognition sites for various genes are provided below.

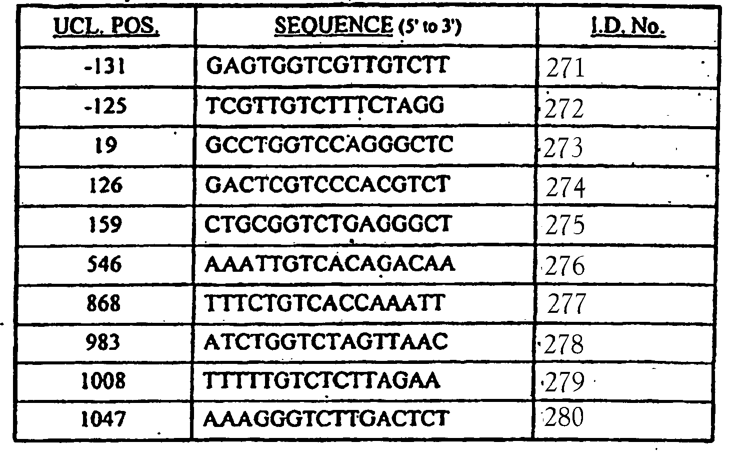

**Table 2**

| TARGET SITE | I.D. No. |
|---|---|
| PCNA HH ribozyme binding sites: | 1-261 |
| Example chimeric hairpin ribozymes: | 262-265 |

### EXAMPLE 2

### CONSTRUCTION OF HAMMERHEAD RIBOZYMES

Chimeric hammerhead ribozymes (i.e., RNA/DNA hybrids) are designed to have an appropriate NUH sequence for ribozyme cleavage. Ribozymes are chemically synthesized with the general structure shown in figure 1. The binding arms bases and stem loop comprise DNA, and the catalytic domain comprises RNA and/or 2'O methyl RNA bases. Specific examples of synthetic human hammerhead ribozymes targeting PCNA are shown below (DNA bases shown in upper case, RNA bases as lower case, 2' O methyl RNA as lower case italics, and propanediol shown as pr pr pr pr):

Alteration of the base composition at the stem loop and catalytic domain increases the catalytic activity of the chimeric ribozyme as assayed by in vitro cleavage (EXAMPLE 3). The substitution of 2' O methyl bases for RNA bases enhances the stability of the chimeric ribozymes. The assay consists of incubating 10 µg of ribozyme with 100 µl of cell lysate at 37°C for times ranging from 30 seconds to 240 minutes, then separating the intact ribozyme from degradation products on a 15% PAGE, staining with SYBRgreen (Molecular Probes, Eugene, OR), and quantifying by phosphorimager analysis (Molecular Dynamics).

By making specific base modifications to the structure of the ribozymes, the half-life in cell lysate was increased sequentially from, approximately 2.5 hours for PN30003, to 3.5 hours for PN30004, and to greater than 10 hours for PN30005 (figure 2). In serum, the half-life of PN30003 is less than 30 seconds. Specific base modifications to ribozyme PN30005 increased the half-life in serum to greater than 4 hours (figure 3). The internal stem loop structure can be substituted with a propanediol linkage to shorten the molecule, facilitating delivery, and enhancing catalytic activity (figure 4).

A serambled sequence polynucleotide including the same composition of ribonucleotides and deoxyribonucleotides is also synthesized for each ribozyme to serve as a control with no catalytic activity. Lipofectin may be utilized to enhance the uptake of ribozyme into the cells.

### EXAMPLE 3

### IN VITRO CLEAVAGE ASSAYS

Hammerhead ribozymes are tested for cleavage activity in an in vitro assay. Ribozyme and substrate synthesis is achieved by a new method of plasmid-independent in vitro transcription (Welch et al 1997). Briefly, oligonucleotides are synthesized (Retrogen, San Diego CA) with the T7 RNA polymerase promoter sequence contiguous with the ribozyme or substrate sequences, to allow for in vitro transcription of annealed oligonucleotides without the need for plasmid cloning. In vitro cleavage is tested in two hour time course reactions in 40 mM Tris pH 7.5, 10 mM. MgCl₂, 2 mM spermidine, at 37°C (Welch et al 1997). Reaction products are analyzed by polyacrylamide gel electrophoresis (PAGE) and quantified by phosphorimager analysis (Molecular Dynamics). The Michaelis constant (Kₘ^{app}) and k₂ are determined for each ribozyme by performing single turnover kinetic experiments with ribozyme concentrations of 2-4 nM and substrate concentrations ranging from 2-200 nM, with analysis as above. The Kₘ^{app} and k₂ for the ribozymes is estimated for a Hanes plot with R2 > 0.90. Catalytic efficiency is calculated as k₂/ Kₘ^{app}.

### EXAMPLE 4

### IN VIVO USE OF RIBOZYMES

### A. Experimental Protocols

All animals are treated according to the guidelines of the Public Health Service Policy on Humane Care and Use of Laboratory Animals.

### Rabbit Disease Model

Dutch belted rabbits of approximately 2 kg are acclimatized for about 14 days in the laboratory environment. 0.06 Units of dispase is injected intravitreally in order to induce disease. Proliferative vitreoretinopathy (PVR) is then scored on days 1, 7, 14, 28 and 48 (1 = 0, 5 = retinal detachment). Typical digital pictures of the process are taken by fundoscopy.

The rabbits are treated on day 21 by injection of either ribozyme or control. The study is terminated when the control (vehicle) sites score as fully detached. Treatment group may be continued if detachment not evident.

### B. In vivo results

An example of the use of the PCNA targeted ribozymes formulated in DOTAP:cholesterol in the rabbit dispase model is shown in figure 5. Panel A shows the normal eye. Panel B shows the effect of dispase followed by treatment with vehicle after 31 days. Panel C shows the effect of dispase followed by treatment with ribozyme after 31 days. The retina is complete detached in the vehicle treated eye. The extent of proliferative vitreoretinopathy is diminished and no retinal detachment is evident in the ribozyme treated eye.

From the foregoing, it will be appreciated that, although specific embodiments of the invention have been described herein for purposes of illustration, various modifications may be made. Accordingly, the invention is not limited except as by the appended claims.

### EXAMPLE 5

### INHIBITION OF EXPERIMENTAL PVR BY RIBOZYMES TARGETING RABBIT PCNA

### A. Experimental PVR treated with Ribozyme:Lipid Complex

### Experimental design

The rabbit model involves the injection of dispase into the vitreal space without the need to introduce retinal tears. Prior models have relied on exogenous cells such as fibroblasts to induce disease. In the dispase model, no assumptions are made as to the type of endogenous cells involved in the formation of disease. The severity of PVR correlated with the dose of dispase injected. The optimal dose of dispase has been determined to minimize interfering complications such as cataract and hemorrhage. This model uses intravitreal rather than subretinal injection. The timing and complexity of the cellular response correspond to the human disease. These advantages make this model particularly attractive. The single disadvantage of relatively long timeframe is easily overcome by extending the period of observation. This model was used to test the efficacy of the chimeric PCNA targeted ribozyme.

A selected lot of dispase was tested in the rabbit eye to titrate the dose and time frame for disease development and aliquots frozen for all remaining experiments (Frenzel EM, Neely KA, Walsh AW, Cameron JD, Gregerson DS. 1998. A new model of proliferative vitreoretinopathy. IOVS 39:2157-2164). Twelve 1-2 Kg Dutch Belted rabbits received a single intravitreal injection of 0.06, 0.07, or 0.08 U dispase (Boehringer Mannheim, Indianapolis, IN) on day 0 (four rabbits each dose). Rabbits were anesthetized with ketamine/xylazine, their eyes dilated with 2.5% phenylephrine and 1% tropicamide, and a local anesthetic, proparacain HCl applied. 0.1 cc of dispase was injected into the right eye of each rabbit with a 30 gauge needle, delivering the dispase into the vitreous cavity directly in front of the retina without traumatizating the retina, under direct visualization with a dissecting microscope equipped with floating Charles' lens. Left eyes serve as controls. Rabbits were followed for progression of disease by fundoscopy at days 1, 7, 14, 21, 28, and 48. A generous timeframe was allocated to maximize the probability of disease development in the dispase treated groups. Fundoscopic evaluation was by two independent, trained observers for this and the following experiments. In this experiment, a dose of 0.07 U dispase resulted in uniform PVR development by 21 days.

Following the same protocol, a second experiment was performed with eight rabbits given 0.07 U dispase in the right eye on day 0. At 7-14 days post-injection, all eyes had vitreous and pre-retinal hemorrhage. At 21 days 7 of 8 rabbits had early PVR characterized by fibroglial proliferation. Before a retinal detachment was noted in any eyes, the control group of rabbits (n=4) was treated with an intravitreal injection of 0.1 cc DOTAP:cholesterol vehicle. The experimental group (n=4) was given an intravitreal injection of 0.1 cc chimeric PCNA targeted ribozyme (PN30006).

### Fundoscopic analysis

Fundoscopic evaluation of chimeric PCNA targeted ribozyme versus control treated rabbit eyes is the same as shown in Figure 5. By day 35, all rabbits in the control group developed significant retinal detachment. Three of four in the chimeric PCNA targeted ribozyme treated group did not develop a retinal detachment, while one developed a small local detachment, which did not progress over the 60 day follow-up period. These data suggest that inhibiting proliferation of cells causative of PVR with chimeric ribozyme to PCNA may have a therapeutic or preventative role.

All eyes were scored by two independent trained observers on a scale of 1-6 as per Table 3 (Fastenberg et al. 1982. A comparison of different cellular inocula in an experimental model of massive periretinal proliferation. Am J Ophthalmol. 93:559-56; Sakamoto et al. 1995. Inhibition of experimental proliferative vitreoretinopathy by retroviral vector-mediated transfer of suicide gene. Ophthalmology 102:1417-1424). The scale is based on inflammation, hemorrhage, distortion, intravitreal haze and membranes, and formation of retinal breaks, traction, and detachments. The results (Figure 6) indicate that the chimeric ribozyme is effective in preventing or retarding the development of PVR.

**Table 3 Histopathologic scoring of PVR**

| Score | Histopathologic Evaluation |
|---|---|
| 0 | Normal eye |
| 1 | Hemorrhage/preretinal gliotic changes |
| 2 | Focal Retration |
| 3 | Extensive PVR with focal traction |
| 4 | PVR with focal retinal detachments of medullary ray |
| 5 | Extensive peripapillary retinal detachments |
| 6 | Total retinal detachments |

### Histopathologic analysis

Treated and control eyes were enucleated, fixed in 4% paraformaldehyde, embedded in paraffin, and sectioned for histopathologic evaluation. Selected gross specimens were photographed before sectioning. Histologic examination was performed after hematoxylin and eosin staining. Histopathologic analyses correlated with the gross pathological findings and confirmed the clinical diagnoses of PVR and retinal detachment.

The ability of this chimeric ribozyme complex to prevent or lessen the degree of damage due to aberrant proliferation of cells in the eye could be a significant advancement in the treatment of PVR. This type of therapy may be especially well suited for use in patients facing vision loss due to the failure of traditional surgical treatment (McCormack P, Simcock PR, Charteris D, Lavin MJ. 1994. Is surgery for proliferative vitreoretinopathy justifiable? Eye 8:75-76.).

### B. Experimental PVR treated with Ribozyme Alone

Three additional experiments in the PVR model were carried out. Experiment 2 was a repeat of the first experiment described above (*i.e.*, Example 5, Section A) with the addition of a control group with scrambled sequence (random specificity) ribozyme complexed to the lipid. The other two experiments were done using the ribozyme alone at 10 mg/ml without the addition of the DOTAP:cholesterol lipid. These were done due to an observed precipitate that took greater than 2 weeks to clear. The precipitate was not observed when lipid was absent. Experiment 3 repeated the timing of the first two experiments, with ribozyme treatment on day 20. Experiment 4 was performed with treatment on day 7 to represent a prophylactic approach. The overall severity of the PVR has decreased over time even in the control group consistent with loss of activity of the dispase. The results are shown in table 4 where two independent assessments were made by trained observers using a modified Fastenberg et al. (1982) scoring system *(i.e.,* Table 3).

**Table 4 Scoring of ribozyme and control treated rabbit eyes with Dispase induced PVR.**

| Exp # | | Ribozyme | Lipid | N | Treatment Day | Assessment Day | Ave. Score |
|---|---|---|---|---|---|---|---|
| 2 | Treated | PN30004 | + | 4 | 20 | 35 | 3.1±1.3 |
| | Control | Scrambled | | 4 | | | 5.3±1.2 |
| | | Dextrose | | 4 | | | 4.5±1 |
| 3 | Treated | PN30006 | - | 4 | 20 | 49 | 1.8±1.7 |
| | Control | Dextrose | | 3 | | | 3.7±0.6 |
| | | no injection | | 2 | | | 2.5±0.7 |
| 4 | Treated | PN30006 | - | 6 | 7 | 28 | 0.7±0.8 |
| | Control | Dextrose | | 6 | | | 2.2±0.4 |

The results show a consistent decrease in severity of PVR following treatment with active chimeric PCNA targeted ribozyme compared to untreated or scrambled ribozyme controls.

### SEQUENCE LISTING

<110> Immusol Incorporated
<120> RIBOZYME THERAPY FOR THE TREATMENT OF PROLIFERATIVE SKIN AND EYE DISEASES
<130> SMW/FP6046981
<140> EPA 00975399.7
   <141> 2000-10-26
<150> PCT/US00/29500
   <151> 2000-10-26
<150> US
   <151> 2000-10-25
<160> 282
<170> PatentIn Ver. 2.0
<210> 1
   <211> 19
   <212> DNA
   <213> Homo sapiens
<220>
   <223> PCNA HH ribozyme binding site
<400> 1
   ttaaacggtt gcaggcgta 19
<210> 2<211> 19
   <212> DNA
   <213> Homo sapiens
<220>
   <223> PCNA HH ribozyme binding site
<400> 2
   tgcaggcgta gagagtggt 19
<210> 3
   <211> 19
   <212> DNA
   <213> Homo sapiens
<220>
   <223> PCNA HH ribozyme binding site
<400> 3
   gagagtggtc gttgtcttt 19
<210> 4<211> 19
   <212> DNA
   <213> Homo sapiens
<220>
<223> PCNA HH ribozyme binding site
<400> 4
   agtggtcgtt gtctttcta 19
<210> 5
   <211> 19
   <212> DNA
   <213> Homo sapiens
<220>
   <223> PCNA HH ribozyme binding site
<400> 5
   ggtcgttgtc tttctaggt 19
<210> 6
   <211> 19
   <212> DNA
   <213> Homo sapiens
<220>
   <223> PCNA HH ribozyme binding site
<400> 6
   tcgttgtctt tctaggtct 19
   <210> 7
   <211> 19
   <212> DNA
   <213> Homo sapiens
<220>
   <223> PCNA HH ribozyme binding site
<400> 7
   cgttgtcttt ctaggtctc 19
<210> 8
   <211> 19
   <212> DNA
   <213> Homo sapiens
<220>
   <223> PCNA HH ribozyme binding site
<400> 8
   gttgtctttc taggtctca 19
<210> 9
   <211> 19
   <212> DNA
   <213> Homo sapiens
<220>
   <223> PCNA HH ribozyme binding site
<400> 9
   tgtctttcta ggtctcagc 19
<210> 10<211> 19
   <212> DNA
   <213> Homo sapiens
<220>
   <223> PCNA HH ribozyme binding site
<400> 10
   tttctaggtc tcagccggt 19
<210> 11
   <211> 19
   <212> DNA
   <213> Homo sapiens
<220>
   <223> PCNA HH ribozyme binding site
<400> 11
   tctaggtctc agccggtcg 19
<210> 12
   <211> 19
   <212> DNA
   <213> Homo sapiens
<220>
   <223> PCNA HH ribozyme binding site
<400> 12
   tcagccggtc gtcgcgacg 19
<210> 13
   <211> 19
   <212> DNA
   <213> Homo sapiens
<220>
   <223> PCNA HH ribozyme binding site
<400> 13
   gccggtcgtc gcgacgttc 19
<210> 14
   <211> 19
   <212> DNA
   <213> Homo sapiens
<220>
   <223> PCNA HH ribozyme binding site
<400> 14
   tcgcgacgtt cgcccgctc 19
<210> 15
   <211> 19
   <212> DNA
   <213> Homo sapiens
<220>
   <223> PCNA HH ribozyme binding site
<400> 15
   cgcgacgttc gcccgctct 19
<210> 16
   <211> 19
   <212> DNA
   <213> Homo sapiens
<220>
   <223> PCNA HH ribozyme binding site
<400> 16
   tcgcccgctc tgaggctcc 19
<210> 17
   <211> 19
   <212> DNA
   <213> Homo sapiens
<220>
   <223> PCNA HH ribozyme binding site
<400> 17
   tctgaggctc ctgaagccg 19
<210> 18
   <211> 19
   <212> DNA
   <213> Homo sapiens
<220>
   <223> PCNA HH ribozyme binding site
<400> 18
   gccgaaacta gctagactt 19
<210> 19
   <211> 19
   <212> DNA
   <213> Homo sapiens
<220>
   <223> PCNA HH ribozyme binding site
<400> 19
   aaactagcta gactttcct 19
<210> 20
   <211> 19
   <212> DNA
   <213> Homo sapiens
<220>
   <223> PCNA HH ribozyme binding site
<400> 20
   agctagactt tcctccttc 19
<210> 21
   <211> 19
   <212> DNA
<213> Homo sapiens
<220>
   <223> PCNA HH ribozyme binding site
<400> 21.
   gctagacttt cctccttcc 19
<210> 22
   <211> 19
   <212> DNA
   <213> Homo sapiens
<220>
   <223> PCNA HH ribozyme binding site
<400> 22
   ctagactttc ctccttccc 19
<210> 23
   <211> 19
   <212> DNA
   <213> Homo sapiens
<220>
   <223> PCNA HH ribozyme binding site
<400> 23
   gactttcctc cttcccgcc 19
<210> 24
   <211> 19
   <212> DNA
   <213> Homo sapiens
<220>
   <223> PCNA HH ribozyme binding site
<400> 24
   tttcctcctt cccgcctgc 19
<210> 25
   <211> 19
   <212> DNA
   <213> Homo sapiens
<220>
   <223> PCNA HH ribozyme binding site
<400> 25
   ttcctccttc ccgcctgcc 19
<210> 26
   <211> 19
   <212> DNA
   <213> Homo sapiens
<220>
   <223> PCNA HH ribozyme binding site
<400> 26
   cctgcctgta gcggcgttg 19
<210> 27
   <211> 19
   <212> DNA
   <213> Homo sapiens
<220>
   <223> PCNA HH ribozyme binding site
<400> 27
   tagcggcgtt gccactccg 19
<210> 28
   <211> 19
   <212> DNA
   <213> Homo sapiens
<220>
   <223> PCNA HH ribozyme binding site
<400> 28
   gttgccactc cgccaccat 19
<210> 29<211> 19
   <212> DNA
   <213> Homo sapiens
<220>
   <223> PCNA HH ribozyme binding site
<400> 29
   ccaccatgtt cgaggcgcg 19
<210> 30
   <211> 19
   <212> DNA
   <213> Homo sapiens
<220>
   <223> PCNA HH ribozyme binding site
<400> 30
   caccatgttc gaggcgcgc 19
<210> 31<211> 19
   <212> DNA
   <213> Homo sapiens
<220>
   <223> PCNA HH ribozyme binding site
<400> 31
   gcgcctggtc cagggctcc 19
<210> 32
   <211> 19
   <212> DNA
   <213> Homo sapiens
<220>
   <223> PCNA HH ribozyme binding site
<400> 32
   tccagggctc catcctcaa 19
<210> 33
   <211> 19
   <212> DNA
   <213> Homo sapiens
<220>
   <223> PCNA HH ribozyme binding site
<400> 33
   gggctccatc ctcaagaag 19
<210> 34
   <211> 19
   <212> DNA
   <213> Homo sapiens
<220>
   <223> PCNA HH ribozyme binding site
<400> 34
   ctccatcctc aagaaggtg 19
<210> 35
   <211> 19
   <212> DNA
   <213> Homo sapiens
<220>
   <223> PCNA HH ribozyme binding site
<400> 35
   agaaggtgtt ggaggcact 19
<210> 36
   <211> 19
   <212> DNA
   <213> Homo sapiens
<220>
   <223> PCNA HH ribozyme binding site
<400> 36
   ggaggcactc aaggacctc 19
<210> 37
   <211> 19
   <212> DNA
   <213> Homo sapiens
<220>
   <223> PCNA HH ribozyme binding site
<400> 37
   caaggacctc atcaacgag 19
<210> 38
<211> 19
   <212> DNA
   <213> Homo sapiens
<220>
   <223> PCNA HH ribozyme binding site
<400> 38
   ggacctcatc aacgaggcc 19
<210> 39
   <211> 19
   <212> DNA
   <213> Homo sapiens
<220>
   <223> PCNA HH ribozyme binding site
<400> 39
   tgctgggata ttagctcca 19
<210> 40
   <211> 19
   <212> DNA
   <213> Homo sapiens
<220>
   <223> PCNA HH ribozyme binding site
<400> 40
   ctgggatatt agctccagc 19
<210> 41
   <211> 19
   <212> DNA
   <213> Homo sapiens
<220>
   <223> PCNA HH ribozyme binding site
<400> 41
   tgggatatta gctccagcg 19
<210> 42
   <211> 19
   <212> DNA
   <213> Homo sapiens
<220>
   <223> PCNA HH ribozyme binding site
<400> 42
   atattagctc cagcggtgt 19
<210> 43
   <211> 19
   <212> DNA
   <213> Homo sapiens
<220>
   <223> PCNA HH ribozyme binding site
<400> 43
   cagcggtgta aacctgcag 19
<210> 44
   <211> 19
   <212> DNA
   <213> Homo sapiens
<220>
   <223> PCNA HH ribozyme binding site
<400> 44
   gcatggactc gtcccacgt 19
<210> 45
   <211> 19
   <212> DNA
   <213> Homo sapiens
<220>
   <223> PCNA HH ribozyme binding site
<400> 45
   tggactcgtc ccacgtctc 19
<210> 46
   <211> 19
   <212> DNA
   <213> Homo sapiens
<220>
   <223> PCNA HH ribozyme binding site
<400> 46
   gtcccacgtc tctttggtg 19
<210> 47
   <211> 19
   <212> DNA
   <213> Homo sapiens
<220>
   <223> PCNA HH ribozyme binding site
<400> 47
   cccacgtctc tttggtgca 19
<210> 48<211> 19
   <212> DNA
   <213> Homo sapiens
<220>
   <223> PCNA HH ribozyme binding site
<400> 48cacgtctctt tggtgcagc
<210> 49
   <211> 19
   <212> DNA
<213> Homo sapiens
<220>
   <223> PCNA HH ribozyme binding site
<400> 49
   acgtctcttt ggtgcagct 19
<210> 50
   <211> 19
   <212> DNA
   <213> Homo sapiens
<220>
   <223> PCNA HH ribozyme binding site
<400> 50
   ggtgcagctc accctgcgg 19
<210> 51
   <211> 19
   <212> DNA
   <213> Homo sapiens
<220>
   <223> PCNA HH ribozyme binding site
<400> 51
   ccctgcggtc tgagggctt 19
<210> 52
   <211> 19
   <212> DNA
   <213> Homo sapiens
<220>
   <223> PCNA HH ribozyme binding site
<400> 52
   ctgagggctt cgacaccta 19
<210> 53
   <211> 19
   <212> DNA
   <213> Homo sapiens
<220>
   <223> PCNA HH ribozyme binding site
<400> 53
   tgagggcttc gacacctac 19
<210> 54
   <211> 19
   <212> DNA
   <213> Homo sapiens
<220>
   <223> PCNA HH ribozyme binding site
<400> 54
   tcgacaccta ccgctgcga 19
<210> 55
   <211> 19
   <212> DNA
   <213> Homo sapiens
<220>
   <223> PCNA HH ribozyme binding site
<400> 55
   cgtgaacctc accagtatg 19
<210> 56
   <211> 19
   <212> DNA
   <213> Homo sapiens
<220>
   <223> PCNA HH ribozyme binding site
<400> 56
   ctcaccagta tgtccaaaa 19
<210> 57<211> 19
   <212> DNA
   <213> Homo sapiens
<220>
   <223> PCNA HH ribozyme binding site
<400> 57
   ccagtatgtc caaaatact 19
<210> 58
   <211> 19
   <212> DNA
   <213> Homo sapiens
<220>
   <223> PCNA HH ribozyme binding site
<400> 58
   gtccaaaata ctaaaatgc 19
<210> 59
   <211> 19
   <212> DNA
   <213> Homo sapiens
<220>
   <223> PCNA HH ribozyme binding site
<400> 59
   caaaatacta aaatgcgcc 19
<210> 60
   <211> 19
   <212> DNA
   <213> Homo sapiens
<220>
   <223> PCNA HH ribozyme binding site
<400> 60
   aatgaagata tcattacac 19
<210> 61
   <211> 19
   <212> DNA
   <213> Homo sapiens
<220>
   <223> PCNA HH ribozyme binding site
<400> 61tgaagatatc attacacta
<210> 62<211> 19
   <212> DNA
   <213> Homo sapiens
<220>
   <223> PCNA HH ribozyme binding site
<400> 62
   agatatcatt acactaagg 19
<210> 63
   <211> 19
   <212> DNA
   <213> Homo sapiens
<220>
   <223> PCNA HH ribozyme binding site
<400> 63
   gatatcatta cactaaggg 19
<210> 64<211> 19
   <212> DNA
   <213> Homo sapiens
<220>
   <223> PCNA HH ribozyme binding site
<400> 64
   cattacacta agggccgaa 19
<210> 65
   <211> 19
   <212> DNA
   <213> Homo sapiens
<220>
   <223> PCNA HH ribozyme binding site
<400> 65
   gccgaagata acgcggata 19
<210> 66
   <211> 19
   <212> DNA
   <213> Homo sapiens
<220>
   <223> PCNA HH ribozyme binding site
<400> 66
   aacgcggata ccttggcgc 19
<210> 67
   <211> 19
   <212> DNA
   <213> Homo sapiens
<220>
   <223> PCNA HH ribozyme binding site
<400> 67
   cggatacctt ggcgctagt 19
<210> 68
   <211> 19
   <212> DNA
   <213> Homo sapiens
<220>
   <223> PCNA HH ribozyme binding site
<400> 68
   cttggcgcta gtatttgaa 19
<210> 69
   <211> 19
   <212> DNA
   <213> Homo sapiens
<220>
   <223> PCNA HH ribozyme binding site
<400> 69
   ggcgctagta tttgaagca 19
<210> 70
   <211> 19
   <212> DNA
   <213> Homo sapiens
<220>
   <223> PCNA HH ribozyme binding site
<400> 70
   cgctagtatt tgaagcacc 19
<210> 71
   <211> 19
   <212> DNA
   <213> Homo sapiens
<220>
   <223> PCNA HH ribozyme binding site
<400> 71
   gctagtattt gaagcacca 19
<210> 72
   <211> 19
   <212> DNA
   <213> Homo sapiens
<220>
   <223> PCNA HH ribozyme binding site
<400> 72
   ggagaaagtt tcagactat 19
<210> 73
   <211> 19
   <212> DNA
   <213> Homo sapiens
<220>
   <223> PCNA HH ribozyme binding site
<400> 73
   gagaaagttt cagactatg 19
<210> 74
   <211> 19
   <212> DNA
   <213> Homo sapiens
<220>
   <223> PCNA HH ribozyme binding site
<400> 74
   agaaagtttc agactatga 19
<210> 75
   <211> 19
   <212> DNA
   <213> Homo sapiens
<220>
   <223> PCNA HH ribozyme binding site
<400> 75
   tttcagacta tgaaatgaa 19
<210> 76
   <211> 19
   <212> DNA
   <213> Homo sapiens
<220>
   <223> PCNA HH ribozyme binding site
<400> 76
   aaatgaagtt gatggattt 19
<210> 77
   <211> 19
   <212> DNA
   <213> Homo sapiens
<220>
   <223> PCNA HH ribozyme binding site
<400> 77
   ttgatggatt tagatgttg 19
<210> 78
   <211> 19
   <212> DNA
   <213> Homo sapiens
<220>
   <223> PCNA HH ribozyme binding site
<400> 78
   tgatggattt agatgttga 19
<210> 79
   <211> 19
   <212> DNA
   <213> Homo sapiens
<220>
   <223> PCNA HH ribozyme binding site
<400> 79
   gatggattta gatgttgaa 19
<210> 80
   <211> 19
   <212> DNA
   <213> Homo sapiens
<220>
   <223> PCNA HH ribozyme binding site
<400> 80
   tttagatgtt gaacaactt 19
<210> 81
   <211> 19
   <212> DNA
   <213> Homo sapiens
<220>
   <223> PCNA HH ribozyme binding site
<400> 81
   tgaacaactt ggaattcca 19
<210> 82
   <211> 19
   <212> DNA
   <213> Homo sapiens
<220>
   <223> PCNA HH ribozyme binding site
<400> 82
   acttggaatt ccagaacag 19
<210> 83
   <211> 19
   <212> DNA
   <213> Homo sapiens
<220>
   <223> PCNA HH ribozyme binding site
<400> 83
   cttggaattc cagaacagg 19
<210> 84
   <211> 19
   <212> DNA
   <213> Homo sapiens
<220>
   <223> PCNA HH ribozyme binding site
<400> 84
   aacaggagta cagctgtgt 19
<210> 85
   <211> 19
   <212> DNA
   <213> Homo sapiens
<220>
   <223> PCNA HH ribozyme binding site
<400> 85
   cagctgtgta gtaaagatg 19
<210> 86
   <211> 19
   <212> DNA
   <213> Homo sapiens
<220>
   <223> PCNA HH ribozyme binding site
<400> 86
   ctgtgtagta aagatgcct 19
<210> 87
   <211> 19
   <212> DNA
   <213> Homo sapiens
<220>
   <223> PCNA HH ribozyme binding site
<400> 87
   aagatgcctt ctggtgaat 19
<210> 88
   <211> 19
   <212> DNA
   <213> Homo sapiens
<220>
   <223> PCNA HH ribozyme binding site
<400> 88
   agatgccttc tggtgaatt 19
<210> 89
   <211> 19
   <212> DNA
   <213> Homo sapiens
<220>
   <223> PCNA HH ribozyme binding site
<400> 89
   ctggtgaatt tgcacgtat 19
<210> 90
   <211> 19
   <212> DNA
   <213> Homo sapiens
<220>
   <223> PCNA HH ribozyme binding site
<400> 90
   tggtgaattt gcacgtata 19
<210> 91
   <211> 19
   <212> DNA
   <213> Homo sapiens
<220>
   <223> PCNA HH ribozyme binding site
<400> 91
   tttgcacgta tatgccgag 19
<210> 92
   <211> 19
   <212> DNA
   <213> Homo sapiens
<220>
   <223> PCNA HH ribozyme binding site
<400> 92
   tgcacgtata tgccgagat 19
<210> 93
   <211> 19
   <212> DNA
   <213> Homo sapiens
<220>
   <223> PCNA HH ribozyme binding site
<400> 93
   tgccgagatc tcagccata 19
<210> 94
   <211> 19
   <212> DNA
   <213> Homo sapiens
<220>
   <223> PCNA HH ribozyme binding site
<400> 94
   ccgagatctc agccatatt 19
<210> 95
   <211> 19
   <212> DNA
   <213> Homo sapiens
<220>
   <223> PCNA HH ribozyme binding site
<400> 95
   ctcagccata ttggagatg 19
<210> 96
   <211> 19
   <212> DNA
   <213> Homo sapiens
<220>
   <223> PCNA HH ribozyme binding site
<400> 96
   cagccatatt ggagatgct 19
<210> 97
   <211> 19
   <212> DNA
   <213> Homo sapiens
<220>
   <223> PCNA HH ribozyme binding site
<400> 97
   agatgctgtt gtaatttcc 19
<210> 98
   <211> 19
   <212> DNA
   <213> Homo sapiens
<220>
   <223> PCNA HH ribozyme binding site
<400> 98
   tgctgttgta atttcctgt 19
<210> 99
   <211> 19
   <212> DNA
   <213> Homo sapiens
<220>
   <223> PCNA HH ribozyme binding site
<400> 99
   tgttgtaatt tcctgtgca 19
<210> 100
   <211> 19
   <212> DNA
   <213> Homo sapiens
<220>
   <223> PCNA HH ribozyme binding site
<400> 100
   gttgtaattt cctgtgcaa 19
<210> 101
   <211> 19
   <212> DNA
   <213> Homo sapiens
<220>
   <223> PCNA HH ribozyme binding site
<400> 101
   ttgtaatttc ctgtgcaaa 19
<210> 102
   <211> 19
   <212> DNA
   <213> Homo sapiens
<220>
   <223> PCNA HH ribozyme binding site
<400> 102
   gagtgaaatt ttctgcaag 19
<210> 103<211> 19
   <212> DNA
   <213> Homo sapiens
<220>
   <223> PCNA HH ribozyme binding site
<400> 103
   agtgaaattt tctgcaagt 19
<210> 104
   <211> 19
   <212> DNA
   <213> Homo sapiens
<220>
   <223> PCNA HH ribozyme binding site
<400> 104
   gtgaaatttt ctgcaagtg 19
   <210> 105
   <211> 19
   <212> DNA
   <213> Homo sapiens
<220>
   <223> PCNA HH ribozyme binding site
<400> 105
   tgaaattttc tgcaagtgg 19
<210> 106
   <211> 19
   <212> DNA
   <213> Homo sapiens
<220>
   <223> PCNA HH ribozyme binding site
<400> 106
   tggagaactt ggaaatgga 19
<210> 107
   <211> 19
   <212> DNA
   <213> Homo sapiens
<220>
   <223> PCNA HH ribozyme binding site
<400> 107
   tggaaacatt aaattgtca 19
<210> 108<211> 19
   <212> DNA
   <213> Homo sapiens
<220>
   <223> PCNA HH ribozyme binding site
<400> 108
   ggaaacatta aattgtcac 19
   <210> 109
   <211> 19
   <212> DNA
   <213> Homo sapiens
<220>
   <223> PCNA HH ribozyme binding site
<400> 109
   acattaaatt gtcacagac 19
   <210> 110
   <211> 19
   <212> DNA
   <213> Homo sapiens
<220>
   <223> PCNA HH ribozyme binding site
<400> 110
   ttaaattgtc acagacaag 19
<210> 111
   <211> 19
   <212> DNA
   <213> Homo sapiens
<220>
   <223> PCNA HH ribozyme binding site
<400> 111
   cagacaagta atgtcgata 19
<210> 112
   <211> 19
   <212> DNA
   <213> Homo sapiens
<220>
   <223> PCNA HH ribozyme binding site
<400> 112
   aagtaatgtc gataaagag 19
<210> 113
   <211> 19
   <212> DNA
   <213> Homo sapiens
<220>
   <223> PCNA HH ribozyme binding site
<400> 113
   aatgtcgata aagaggagg 19
<210> 114
   <211> 19
   <212> DNA
   <213> Homo sapiens
<220>
   <223> PCNA HH ribozyme binding site
<400> 114
   ggaagctgtt accatagag 19
<210> 115
   <211> 19
   <212> DNA
   <213> Homo sapiens
<220>
   <223> PCNA HH ribozyme binding site
<400> 115
   gaagctgtta ccatagaga 19
<210> 116
   <211> 19
   <212> DNA
   <213> Homo sapiens
<220>
   <223> PCNA HH ribozyme binding site
<400> 116
   tgttaccata gagatgaat 19
<210> 117
   <211> 19
   <212> DNA
   <213> Homo sapiens
<220>
   <223> PCNA HH ribozyme binding site
<400> 117
   tgaaccagtt caactaact 19
<210> 118
   <211> 19
   <212> DNA
   <213> Homo sapiens
<220>
   <223> PCNA HH ribozyme binding site
<400> 118
   gaaccagttc aactaactt 19
<210> 119
   <211> 19
   <212> DNA
   <213> Homo sapiens
<220>
   <223> PCNA HH ribozyme binding site
<400> 119
   agttcaacta acttttgca 19
<210> 120
   <211> 19
   <212> DNA
   <213> Homo sapiens
<220>
   <223> PCNA HH ribozyme binding site
<400> 120
   caactaactt ttgcactga 19
<210> 121
   <211> 19
   <212> DNA
   <213> Homo sapiens
<220>
   <223> PCNA HH ribozyme binding site
<400> 121
<210> 122 .
   <211> 19
   <212> DNA
   <213> Homo sapiens
<220>
   <223> PCNA HH ribozyme binding site
<400> 122
   actaactttt gcactgagg   19
<210> 123
   <211> 19
   <212> DNA
   <213> Homo sapiens
<220>
   <223> PCNA HH ribozyme binding site
<400> 123
   cactgaggta cctgaactt 19
<210> 124
   <211> 19
   <212> DNA
   <213> Homo sapiens
<220>
   <223> PCNA HH ribozyme binding site
<400> 124
   acctgaactt ctttacaaa 19
<210> 125
   <211> 19
   <212> DNA
   <213> Homo sapiens
<220>
   <223> PCNA HH ribozyme binding site
<400> 125
   cctgaacttc tttacaaaa 19
   <210> 126
   <211> 19
   <212> DNA
   <213> Homo sapiens
<220>
   <223> PCNA HH ribozyme binding site
<400> 126
   tgaacttctt tacaaaagc 19
   <210> 127
   <211> 19
   <212> DNA
   <213> Homo sapiens
<220>
   <223> PCNA HH ribozyme binding site
<400> 127
   gaacttcttt acaaaagcc 19
<210> 128
   <211> 19
   <212> DNA
   <213> Homo sapiens
<220>
   <223> PCNA HH ribozyme binding site
<400> 128
   aacttcttta caaaagcca 19
<210> 129
   <211> 19
   <212> DNA
   <213> Homo sapiens
<220>
   <223> PCNA HH ribozyme binding site
<400>
   aaagccactc cactctctt 19
<210> 130
   <211> 19
   <212> DNA
   <213> Homo sapiens
<220>
   <223> PCNA HH ribozyme binding site
<400> 130
   cactccactc tcttcaacg 19
<210> 131
   <211> 19
   <212> DNA
   <213> Homo sapiens
<220>
   <223> PCNA HH ribozyme binding site
<400> 131
   ctccactctc ttcaacggt 19
<210> 132
   <211> 19
   <212> DNA
   <213> Homo sapiens
<220>
   <223> PCNA HH ribozyme binding site
<400> 132
   ccactctctt caacggtga 19
<210> 133
   <211> 19
   <212> DNA
   <213> Homo sapiens
<220>
   <223> PCNA HH ribozyme binding site
<400> 133
   cactctcttc aacggtgac 19
<210> 134
   <211> 19
   <212> DNA
   <213> Homo sapiens
<220>
   <223> PCNA HH ribozyme binding site
<400> 134
   ggtgacactc agtatgtct 19
<210> 135
   <211> 19
   <212> DNA
   <213> Homo sapiens
<220>
   <223> PCNA HH ribozyme binding site
<400> 135
   acactcagta tgtctgcag 19
<210> 136
   <211> 19
   <212> DNA
   <213> Homo sapiens
<220>
   <223> PCNA HH ribozyme binding site
<400> 136
   tcagtatgtc tgcagatgt 19
<210> 137
   <211> 19
   <212> DNA
   <213> Homo sapiens
<220>
   <223> PCNA HH ribozyme binding site
<400> 137
   tgcagatgta ccccttgtt 19
<210> 138
   <211> 19
   <212> DNA
   <213> Homo sapiens
<220>
   <223> PCNA HH ribozyme binding site
<400> 138
   tgtacccctt gttgtagag 19
<210> 139
   <211> 19
   <212> DNA
   <213> Homo sapiens
<220>
   <223> PCNA HH ribozyme binding site
<400> 139
   accccttgtt gtagagtat 19
<210> 140
   <211> 19
   <212> DNA
   <213> Homo sapiens
<220>
   <223> PCNA HH ribozyme binding site
<400> 140
   ccttgttgta gagtataaa 19
<210> 141
   <211> 19
   <212> DNA
   <213> Homo sapiens
<220>
   <223> PCNA HH ribozyme binding site
<400> 141
   ttgtagagta taaaattgc 19
<210> 142
   <211> 19
   <212> DNA
   <213> Homo sapiens
<220>
   <223> PCNA HH ribozyme binding site
<400> 142
   gtagagtata aaattgcgg 19
<210> 143
   <211> 19
   <212> DNA
   <213> Homo sapiens
<220>
   <223> PCNA HH ribozyme binding site
<400> 143
   gtataaaatt gcggatatg 19
<210> 144<211> 19
   <212> DNA
   <213> Homo sapiens
<220>
   <223> PCNA HH ribozyme binding site
<400> 144
   attgcggata tgggacact 19
<210> 145
   <211> 19
   <212> DNA
   <213> Homo sapiens
<220>
   <223> PCNA HH ribozyme binding site
<400> 145
   tgggacactt aaaatacta 19
<210> 146
   <211> 19
   <212> DNA
   <213> Homo sapiens
<220>
   <223> PCNA HH ribozyme binding site
<400> 146
   gggacactta aaatactac 19
<210> 147
   <211> 19
   <212> DNA
   <213> Homo sapiens
<220>
   <223> PCNA HH ribozyme binding site
<400> 147
   acttaaaata ctacttggc 19
<210> 148
   <211> 19
   <212> DNA
   <213> Homo sapiens
<220>
   <223> PCNA HH ribozyme binding site
<400> 148
   taaaatacta cttggctcc 19
<210> 149
   <211> 19
   <212> DNA
   <213> Homo sapiens
<220>
   <223> PCNA HH ribozyme binding site
<400> 149
   aatactactt ggctcccaa 19
<210> 150
   <211> 19
   <212> DNA
   <213> Homo sapiens
<220>
   <223> PCNA HH ribozyme binding site
<400> 150
   tacttggctc ccaagatcg 19
<210> 151
   <211> 19
   <212> DNA
   <213> Homo sapiens
<220>
   <223> PCNA HH ribozyme binding site
<400> 151
   tcccaagatc gaggatgaa 19
<210> 152
   <211> 19
   <212> DNA
   <213> Homo sapiens
<220>
   <223> PCNA HH ribozyme binding site
<400> 152
   aagaaggatc ttaggcatt 19
<210> 153
   <211> 19
   <212> DNA
   <213> Homo sapiens
<220>
   <223> PCNA HH ribozyme binding site
<400> 153
   gaaggatctt aggcattct 19
<210> 154
   <211> 19
   <212> DNA
   <213> Homo sapiens
<220>
   <223> PCNA HH ribozyme binding site
<400> 154
   aaggatctta ggcattctt 19
<210> 155
   <211> 19
   <212> DNA
   <213> Homo sapiens
<220>
   <223> PCNA HH ribozyme binding site
<400> 155
   cttaggcatt cttaaaatt 19
<210> 156<211> 19
   <212> DNA
   <213> Homo sapiens
<220>
   <223> PCNA HH ribozyme binding site
<400> 156
   ttaggcattc ttaaaattc 19
<210> 157
   <211> 19
   <212> DNA
   <213> Homo sapiens
<220>
   <223> PCNA HH ribozyme binding site
<400> 157
   aggcattctt aaaattcaa 19
<210> 158<211> 19
   <212> DNA
   <213> Homo sapiens
<220>
   <223> PCNA HH ribozyme binding site
<400> 158
   ggcattctta aaattcaag 19
<210> 159
   <211> 19
   <212> DNA
   <213> Homo sapiens
<220>
   <223> PCNA HH ribozyme binding site
<400> 159
   tcttaaaatt caagaaaat 19
<210> 160
   <211> 19
   <212> DNA
   <213> Homo sapiens
<220>
   <223> PCNA HH ribozyme binding site
<400> 160
   cttaaaattc aagaaaata 19
<210> 161<211> 19
   <212> DNA
   <213> Homo sapiens
<220>
   <223> PCNA HH ribozyme binding site
<400> 161
   caagaaaata aaactaagc 19
<210> 162
   <211> 19
   <212> DNA
   <213> Homo sapiens
<220>
   <223> PCNA HH ribozyme binding site
<400> 162
   aataaaacta agctctttg 19
<210> 163
   <211> 19
   <212> DNA
   <213> Homo sapiens
<220>
   <223> PCNA HH ribozyme binding site
<400> 163
   aactaagctc tttgagaac 19
<210> 164
   <211> 19
   <212> DNA
   <213> Homo sapiens
<220>
   <223> PCNA HH ribozyme binding site
<400> 164
   ctaagctctt tgagaactg 19
<210> 165
   <211> 19
   <212> DNA
   <213> Homo sapiens
<220>
   <223> PCNA HH ribozyme binding site
<400> 165
   taagctcttt gagaactgc 19
<210> 166
   <211> 19
   <212> DNA
   <213> Homo sapiens
<220>
   <223> PCNA HH ribozyme binding site
<400> 166
   agaactgctt ctaagatgc 19
<210> 167
   <211> 19
   <212> DNA
   <213> Homo sapiens
<220>
   <223> PCNA HH ribozyme binding site
<400> 197
<210> 168
   <211> 19
   <212> DNA
   <213> Homo sapiens
<220>
   <223> PCNA HH ribozyme binding site
<400> 168
   actgcttcta agatgccag 19
<210> 169
   <211> 19
   <212> DNA
   <213> Homo sapiens
<220>
   <223> PCNA HH ribozyme binding site
<400> 169
   tgccagcata tactgaagt 19
<210> 170
   <211> 19
   <212> DNA
   <213> Homo sapiens
<220>
   <223> PCNA HH ribozyme binding site
<400> 170
   ccagcatata ctgaagtct 19
<210> 171
   <211> 19
   <212> DNA
   <213> Homo sapiens
<220>
   <223> PCNA HH ribozyme binding site
<400> 171
   tactgaagtc ttttctgtc 19
<210> 172
   <211> 19
   <212> DNA
   <213> Homo sapiens
<220>
   <223> PCNA HH ribozyme binding site
<400> 172
   ctgaagtctt ttctgtcac 19
<210> 173
   <211> 19
   <212> DNA
   <213> Homo sapiens
<220>
   <223> PCNA HH ribozyme binding site
<400> 173
   tgaagtcttt tctgtcacc 19
<210> 174
   <211> 19
   <212> DNA
   <213> Homo sapiens
<220>
   <223> PCNA HH ribozyme binding site
<400> 174
   gaagtctttt ctgtcacca 19
<210> 175
   <211> 19
   <212> DNA
   <213> Homo sapiens
<220>
   <223> PCNA HH ribozyme binding site
<400> 175
   aagtcttttc tgtcaccaa 19
<210> 176
   <211> 19
   <212> DNA
   <213> Homo sapiens
<220>
   <223> PCNA HH ribozyme binding site
<400> 176
   cttttctgtc accaaattt 19
<210> 177
   <211> 19
   <212> DNA
   <213> Homo sapiens
<220>
   <223> PCNA HH ribozyme binding site
<400> 177
   tcaccaaatt tgtacctct 19
<210> 178
   <211> 19
   <212> DNA
   <213> Homo sapiens
<220>
   <223> PCNA HH ribozyme binding site
<400> 178
   caccaaattt gtacctcta 19
<210> 179
   <211> 19
   <212> DNA
   <213> Homo sapiens
<220>
   <223> PCNA HH ribozyme binding site
<400> 179
   caaatttgta cctctaagt 19
<210> 180
   <211> 19
   <212> DNA
   <213> Homo sapiens
<220>
   <223> PCNA HH ribozyme binding site
<400> 180
   tttgtacctc taagtacat 19
<210> 181
   <211> 19
   <212> DNA
   <213> Homo sapiens
<220>
   <223> PCNA HH ribozyme binding site
<400> 181
   tgtacctcta agtacatat 19
<210> 182
   <211> 19
   <212> DNA
   <213> Homo sapiens
<220>
   <223> PCNA HH ribozyme binding site
<400> 182
   cctctaagta catatgtag 19
<210> 183
   <211> 19
   <212> DNA
   <213> Homo sapiens
<220>
   <223> PCNA HH ribozyme binding site
<400> 183
   taagtacata tgtagatat 19
<210> 184
   <211> 19
   <212> DNA
   <213> Homo sapiens
<220>
   <223> PCNA HH ribozyme binding site
<400> 184
   tacatatgta gatattgtt 19
<210> 185
   <211> 19
   <212> DNA
   <213> Homo sapiens
<220>
   <223> PCNA HH ribozyme binding site
<400> 185
   tatgtagata ttgttttct 19
<210> 186
   <211> 19
   <212> DNA
   <213> Homo sapiens
<220>
   <223> PCNA HH ribozyme binding site
<400> 186
   tgtagatatt gttttctgt 19
<210> 187
   <211> 19
   <212> DNA
   <213> Homo sapiens
<220>
   <223> PCNA HH ribozyme binding site
<400> 187
   agatattgtt ttctgtaaa 19
<210> 188
   <211> 19
   <212> DNA
   <213> Homo sapiens
<220>
   <223> PCNA HH ribozyme binding site
<400> 188
   gatattgttt tctgtaaat 19
<210> 189
   <211> 19
   <212> DNA
   <213> Homo sapiens
<220>
   <223> PCNA HH ribozyme binding site
<400> 189
   atattgtttt ctgtaaata 19
<210> 190
   <211> 19
   <212> DNA
   <213> Homo sapiens
<220>
   <223> PCNA HH ribozyme binding site
<400> 190
   tattgttttc tgtaaataa 19
<210> 191
   <211> 19
   <212> DNA
   <213> Homo sapiens
<220>
   <223> PCNA HH ribozyme binding site
<400> 191
   gttttctgta aataaccta 19
<210> 192
   <211> 19
   <212> DNA
   <213> Homo sapiens
<220>
   <223> PCNA HH ribozyme binding site
<400> 192
   tctgtaaata acctatttt 19
<210> 193
   <211> 19
   <212> DNA
   <213> Homo sapiens
<220>
   <223> PCNA HH ribozyme binding site
<400> 193
   aaataaccta ttttttttc 19
<210> 194
   <211> 19
   <212> DNA
   <213> Homo sapiens
<220>
   <223> PCNA HH ribozyme binding site
<400> 194
   ataacctatt ttttttctc 19
<210> 195
   <211> 19
   <212> DNA
   <213> Homo sapiens
<220>
   <223> PCNA HH ribozyme binding site
<400> 195
   taacctattt tttttctct 19
<210> 196
   <211> 19
   <212> DNA
   <213> Homo sapiens
<220>
   <223> PCNA HH ribozyme binding site
<400> 196
   aacctatttt ttttctcta 19
<210> 197
   <211> 19
   <212> DNA
   <213> Homo sapiens
<220>
   <223> PCNA HH ribozyme binding site
<400> 197
   acctattttt tttctctat 19
<210> 198
   <211> 19
   <212> DNA
   <213> Homo sapiens
<220>
   <223> PCNA HH ribozyme binding site
<400> 198
   cctatttttt ttctctatt 19
<210> 199
   <211> 19
   <212> DNA
   <213> Homo sapiens
<220>
   <223> PCNA HH ribozyme binding site
<400> 199
<210> 200
   <211> 19
   <212> DNA
   <213> Homo sapiens
<220>
   <223> PCNA HH ribozyme binding site
<400> 200
   tatttttttt ctctattct 19
<210> 201
   <211> 19
   <212> DNA
   <213> Homo sapiens
<220>
   <223> PCNA HH ribozyme binding site
<400> 201
   attttttttc tctattctc 19
<210> 202
   <211> 19
   <212> DNA
   <213> Homo sapiens
<220>
   <223> PCNA HH ribozyme binding site
<400> 202
   tttttttctc tattctctc 19
<210> 203
   <211> 19
   <212> DNA
   <213> Homo sapiens
<220>
   <223> PCNA HH ribozyme binding site
<400> 203
   tttttctcta ttctctcca 19
<210> 204
   <211> 19
   <212> DNA
   <213> Homo sapiens
<220>
   <223> PCNA HH ribozyme binding site
<400> 204
   tttctctatt ctctccaat 19
<210> 205
   <211> 19
   <212> DNA
   <213> Homo sapiens
<220>
   <223> PCNA HH ribozyme binding site
<400> 205
   ttctctattc tctccaatt 19
<210> 206
   <211> 19
   <212> DNA
   <213> Homo sapiens
<220>
   <223> PCNA HH ribozyme binding site
<400> 206
   ctctattctc tccaatttg 19
<210> 207
   <211> 19
   <212> DNA
   <213> Homo sapiens
<220>
   <223> PCNA HH ribozyme binding site
<400> 207
   ctattctctc caatttgtt 19
<210> 207
   <211> 19
   <212> DNA
   <213> Homo sapiens
<220>
   <223> PCNA HH ribozyme binding site
<400> 208
<210> 209
   <211> 19
   <212> DNA
   <213> Homo sapiens
<220>
   <223> PCNA HH ribozyme binding site
<400> 209
   tctccaattt gtttaaaga 19
<210> 210
   <211> 19
   <212> DNA
   <213> Homo sapiens
<220>
   <223> PCNA HH ribozyme binding site
<400> 210
   ccaatttgtt taaagaata 19
<210> 211
   <211> 19
   <212> DNA
   <213> Homo sapiens
<220>
   <223> PCNA HH ribozyme binding site
<400> 211
   caatttgttt aaagaataa 19
<210> 212
   <211> 19
   <212> DNA
   <213> Homo sapiens
<220>
   <223> PCNA HH ribozyme binding site
<400> 212
   aatttgttta aagaataaa 19
<210> 213
   <211> 19
   <212> DNA
   <213> Homo sapiens
<220>
   <223> PCNA HH ribozyme binding site
<400> 213
   ttaaagaata aagtccaaa 19
<210> 214
   <211> 19
   <212> DNA
   <213> Homo sapiens
<220>
   <223> PCNA HH ribozyme binding site
<400> 214
   gaataaagtc caaagtctg 19
<210> 215
   <211> 19
   <212> DNA
   <213> Homo sapiens
<220>
   <223> PCNA HH ribozyme binding site
<400> 215
   gtccaaagtc tgatctggt 19
<210> 216
   <211> 19
   <212> DNA
   <213> Homo sapiens
<220>
   <223> PCNA HH ribozyme binding site
<400> 216
   aagtctgatc tggtctagt 19
<210> 217
   <211> 19
   <212> DNA
   <213> Homo sapiens
<220>
   <223> PCNA HH ribozyme binding site
<400> 217
   tgatctggtc tagttaacc 19
<210> 218
   <211> 19
   <212> DNA
   <213> Homo sapiens
<220>
   <223> PCNA HH ribozyme binding site
<400> 218
   atctggtcta gttaaccta 19
<210> 219
   <211> 19
   <212> DNA
   <213> Homo sapiens
<220>
   <223> PCNA HH ribozyme binding site
<400> 219
   tggtctagtt aacctagaa 19
<210> 220
   <211> 19
   <212> DNA
   <213> Homo sapiens
<220>
   <223> PCNA HH ribozyme binding site
<400> 220
   ggtctagtta acctagaag 19
<210> 221
   <211> 19
   <212> DNA
   <213> Homo sapiens
<220>
   <223> PCNA HH ribozyme binding site
<400> 221
   agttaaccta gaagtattt 19
<210> 222
   <211> 19
   <212> DNA '
   <213> Homo sapiens
<220>
   <223> PCNA HH ribozyme binding site
<400> 222
   cctagaagta tttttgtct 19
<210> 223
   <211> 19
   <212> DNA
   <213> Homo sapiens
<220>
   <223> PCNA HH ribozyme binding site
<400> 223
   tagaagtatt tttgtctct 19
<210> 224
   <211> 19
   <212> DNA
   <213> Homo sapiens
<220>
   <223> PCNA HH ribozyme binding site
<400> 224
   agaagtattt ttgtctctt 19
   <210> 225
   <211> 19
   <212> DNA
   <213> Homo sapiens
<220>
   <223> PCNA HH ribozyme binding site
<400> 225
   gaagtatttt tgtctctta 19
   <210> 226
   <211> 19
   <212> DNA
   <213> Homo sapiens
<220>
   <223> PCNA HH ribozyme binding site
<400> 226
   aagtattttt gtctcttag 19
   <210> 227
   <211> 19
   <212> DNA
   <213> Homo sapiens
<220>
   <223> PCNA HH ribozyme binding site
<400> 227
   tatttttgtc tcttagaaa 19
<210> 228
   <211> 19
   <212> DNA
   <213> Homo sapiens
<220>
   <223> PCNA HH ribozyme binding site
<400> 228
   tttttgtctc ttagaaata 19
<210> 229
   <211> 19
   <212> DNA
   <213> Homo sapiens
<220>
   <223> PCNA HH ribozyme binding site
<400> 229
   tttgtctctt agaaatact 19
<210> 230
   <211> 19
   <212> DNA
   <213> Homo sapiens
<220>
   <223> PCNA HH ribozyme binding site
<400> 230
   ttgtctctta gaaatactt 19
<210> 231
   <211> 19
   <212> DNA
   <213> Homo sapiens
<220>
   <223> PCNA HH ribozyme binding site
<400> 231
   cttagaaata cttgtgatt 19
<210> 232
   <211> 19
   <212> DNA
   <213> Homo sapiens
<220>
   <223> PCNA HH ribozyme binding site
<400> 232
   agaaatactt gtgattttt 19
<210> 233
   <211> 19
   <212> DNA
   <213> Homo sapiens
<220>
   <223> PCNA HH ribozyme binding site
<400> 233
   acttgtgatt tttataata 19
<210> 234
   <211> 19
   <212> DNA
   <213> Homo sapiens
<220>
   <223> PCNA HH ribozyme binding site
<400> 234
   cttgtgattt ttataatac 19
<210> 235
   <211> 19
   <212> DNA
   <213> Homo sapiens
<220>
   <223> PCNA HH ribozyme binding site
<400> 235
   ttgtgatttt tataataca 19
<210> 236
   <211> 19
   <212> DNA
   <213> Homo sapiens
<220>
   <223> PCNA HH ribozyme binding site
<400> 236
   tgtgattttt ataatacaa 19
<210> 237
   <211> 19
   <212> DNA
   <213> Homo sapiens
<220>
   <223> PCNA HH ribozyme binding site
<400> 237
   gtgattttta taatacaaa 19
<210> 238
   <211> 19
   <212> DNA
   <213> Homo sapiens
<220>
   <223> PCNA HH ribozyme binding site
<400> 238
   gatttttata atacaaaag 19
<210> 239
   <211> 19
   <212> DNA
   <213> Homo sapiens
<220>
   <223> PCNA HH ribozyme binding site
<400> 239
   ttttataata caaaagggt 19
<210> 240
   <211> 19
   <212> DNA
   <213> Homo sapiens
<220>
   <223> PCNA HH ribozyme binding site
<400> 240
   caaaagggtc ttgactcta 19
<210> 241
   <211> 19
   <212> DNA
   <213> Homo sapiens
<220>
   <223> PCNA HH ribozyme binding site
<400> 241
   aaagggtctt gactctaaa 19
<210> 242<211> 19
   <212> DNA
   <213> Homo sapiens
<220>
   <223> PCNA HH ribozyme binding site
<400> 242
   gtcttgactc taaatgcag 19
<210> 243
   <211> 19
   <212> DNA
   <213> Homo sapiens
<220>
   <223> PCNA HH ribozyme binding site
<400> 243
   cttgactcta aatgcagtt 19
<210> 244
   <211> 19
   <212> DNA
   <213> Homo sapiens
<220>
   <223> PCNA HH ribozyme binding site
<400> 244
   aaatgcagtt ttaagaagt 19
<210> 245
   <211> 19
   <212> DNA
   <213> Homo sapiens
<220>
   <223> PCNA HH ribozyme binding site
<400> 245
   aatgcagttt taagaagtg 19
<210> 246
   <211> 19
   <212> DNA
   <213> Homo sapiens
<220>
   <223> PCNA HH ribozyme binding site
<400> 246
   atgcagtttt aagaagtgt 19
<210> 247
   <211> 19
   <212> DNA
   <213> Homo sapiens
<220>
   <223> PCNA HH ribozyme binding site
<400> 247
   tgcagtttta agaagtgtt 19
<210> 248
   <211> 19
   <212> DNA
   <213> Homo sapiens
<220>
   <223> PCNA HH ribozyme binding site
<400> 248
   aagaagtgtt tttgaattt 19
<210> 249
   <211> 19
   <212> DNA
   <213> Homo sapiens
<220>
   <223> PCNA HH ribozyme binding site
<400> 249
   agaagtgttt ttgaattta 19
<210> 250
   <211> 19
   <212> DNA
   <213> Homo sapiens
<220>
   <223> PCNA HH ribozyme binding site
<400> 250
   gaagtgtttt tgaatttaa 19
<210> 251
   <211> 19
   <212> DNA
   <213> Homo sapiens
<220>
   <223> PCNA HH ribozyme binding site
<400> 251
   aagtgttttt gaatttaaa 19
<210> 252
   <211> 19
   <212> DNA
   <213> Homo sapiens
<220>
   <223> PCNA HH ribozyme binding site
<400> 252
   tttttgaatt taaataaag 19
<210> 253
   <211> 19
   <212> DNA
   <213> Homo sapiens
<220>
   <223> PCNA HH ribozyme binding site
<400> 253
   ttttgaattt aaataaagt 19
<210> 254
   <211> 19
   <212> DNA
   <213> Homo sapiens
<220>
   <223> PCNA HH ribozyme binding site
<400> 254
   tttgaattta aataaagtt 19
<210> 255
   <211> 19
   <212> DNA
   <213> Homo sapiens
<220>
   <223> PCNA HH ribozyme binding site
<400> 255
   aatttaaata aagttactt 19
<210> 256
   <211> 19
   <212> DNA
   <213> Homo sapiens
<220>
   <223> PCNA HH ribozyme binding site
<400> 256
   aaataaagtt acttgaatt 19
<210> 257
   <211> 19
   <212> DNA
   <213> Homo sapiens
<220>
   <223> PCNA HH ribozyme binding site
<400> 257
   aataaagtta cttgaattt 19
<210> 258
   <211> 19
   <212> DNA
   <213> Homo sapiens
<220>
   <223> PCNA HH ribozyme binding site
<400> 258
   aaagttactt gaatttcaa 19
<210> 259
   <211> 19
   <212> DNA
   <213> Homo sapiens
<220>
   <223> PCNA HH ribozyme binding site
<400> 259
   tacttgaatt tcaaacaaa 19
<210> 260
   <211> 19
   <212> DNA
   <213> Homo sapiens
<220>
   <223> PCNA HH ribozyme binding site
<400> 260
   acttgaattt caaacaaaa 19
<210> 261
   <211> 19
   <212> DNA
   <213> Homo sapiens
<220>
   <223> PCNA HH ribozyme binding site
<400> 261
   cttgaatttc aaacaaaaa 19
<210> 262
   <211> 52
   <212> DNA
   <213> Homo sapiens
<400> 262
   gacccctgag aaaggcacca gagaaacaca cttggtggta tattacctgg ta 52
<210> 263
   <211> 52
   <212> DNA
   <213> Homo sapiens
<400> 263
   gagccctgag aaaggcacca gagaaacaca cttggtggta tattacctgg ta 52
<210> 264
   <211> 52
   <212> DNA
   <213> Homo sapiens
<400> 264
   gagccctgag aaaggcacca gcgaaacaca cttggtggca tattacctgg ta 52
<210> 265<211> 61
   <212> DNA
   <213> Homo sapiens
<400> 265
   gagccctgag aaaggcacca gcgaaacaca cggacuucgg tccgtggcat attacctggt 60
   a 61
<210> 266
   <211> 14 _
   <212> DNA
   <213> Homo sapiens
<220>
   <223> General Hairpin Ribozyme Site
<220>
   <221> modified_base
   <222> (1)
   <223> Where n is a, c, g or u
<220>
   <221> modified_base
   <222> (2)
   <223> Where n is a, c, g or u
<220>
   <221> modified_base
   <222> (3)
   <223> Where n is a, c, g or u
<220>
   <221> modified_base
   <222> (5)
   <223> Where n is a, c, g or u
<220>
   <221> modified_base
   <222> (9)
   <223> Where n is a, c, g or u
<220>
   <221> modified_base
   <222> (10)
   <223> Where n is a, c, g or u
<220>
   <221> modified_base
   <222> (11)
   <223> Where n is a, c, g or u
<220>
   <221> modified_base
   <222> (12)
   <223> Where n is a, c, g or u
<220>
   <221> modified_base
   <222> (13)
   <223> Where n is a, c, g or u
<220>
   <221> modified_base
   <222> (14)
   <223> Where n is a, c, g or u
<400> 266
   nnnbngucnn nnnn 14
<210> 267
   <211> 15
   <212> DNA
   <213> Homo sapiens
<220>
   <223> General Hairpin Ribozyme Site
<220>
   <221> modified_base
   <222> (1)
   <223> Where n is a, c, g or u
<220>
   <221> modified_base
   <222> (2)
   <223> Where n is a, c, g or u
<220>
   <221> modified_base
   <222> (3)
   <223> Where n is a, c, g or u
<220>
   <221> modified_base
   <222> (5)
   <223> Where n is a, c, g or u
<220>
   <221> modified_base
   <222> (9)
   <223> Where n is a, c, g or u
<220>
   <221> modified_base
   <222> (10)
   <223> Where n is a, c, g or u
<220>
   <221> modified_base
   <222> (11)
   <223> Where n is a, c, g or u
<220>
   <221> modified_base
   <222> (12)
   <223> Where n is a, c, g or u
<220>
   <221> modified_base
   <222> (13)
   <223> Where n is a, c, g or u
<220>
   <221> modified_base
   <222> (14)
   <223> Where n is a, c, g or u
<220>
   <221> modified_base
   <222> (15)
   <223> Where n is a, c, g or u
<400> 267
   nnnbngucnn nnnnn 15
<210> 268
   <211> 16
   <212> DNA
   <213> Homo sapiens
<220>
   <223> General Hairpin Ribozyme Site
<220>
   <221> modified_base
   <222> (1)
   <223> Where n is a, c, g or u
<220>
   <221> modified_base
   <222> (2)
   <223> Where n is a, c, g or u
<220>
   <221> modified_base
   <222> (3)
   <223> Where n is a, c, g or u
<220>
   <221> modified_base
   <222> (5)
   <223> Where n is a, c, g or u
<220>
   <221> modified_base
   <222> (9)
   <223> Where n is a, c, g or u
<220>
   <221> modified_base
   <222> (10)
   <223> Where n is a, c, g or u
<220>
   <221> modified_base
   <222> (11)
   <223> Where n is a, c, g or u
<220>
   <221> modified_base
   <222> (12)
   <223> Where n is a, c, g or u
<220>
   <221> modified_base
   <222> (13)
   <223> Where n is a, c, g or u
<220>
   <221> modified_base
   <222> (14)
   <223> Where n is a, c, g or u
<220>
   <221> modified_base
   <222> (15)
   <223> Where n is a, c, g or u
<220>
   <221> modified_base
   <222> (16)
   <223> Where n is a, c, g or u
<400> 268
   nnnbngucnn nnnnnn 16
   <210> 269
   <211> 17
   <212> DNA
   <213> Homo sapiens
<220>
   <223> General Hairpin Ribozyme Site
<220>
   <221> modified_base
   <222> (1)
   <223> Where n is a, c, g or u
<220>
   <221> modified_base
   <222> (2)
   <223> Where n is a, c, g or u
<220>
   <221> modified_base
   <222> (3)
   <223> Where n is a, c, g or u
<220>
   <221> modified_base
   <222> (5)
   <223> Where n is a, c, g or u
<220>
   <221> modified_base
   <222> (9)
   <223> Where n is a, c, g or u
<220>
   <221> modified_base
   <222> (10)
   <223> Where n is a, c, g or u
<220>
   <221> modified base
   <222> (11)
   <223> Where n is a, c, g or u
<220>
   <221> modified_base
   <222> (12)
   <223> Where n is a, c, g or u
<220>
   <221> modified_base
   <222> (13)
   <223> Where n is a, c, g or u
<220>
   <221> modified_base
   <222> (14)
   <223> Where n is a, c, g or u
<220>
   <221> modified_base
   <222> (15)
   <223> Where n is a, c, g or u
<220>
   <221> modified_base
   <222> (16)
   <223> Where n is a, c, g or u
<220>
   <221> modified_base
   <222> (17)
   <223> Where n is a, c, g or u
<400> 269
   nnnbngucnn nnnnnnn 17
<210> 270
   <211> 18
   <212> DNA
   <213> Homo sapiens
<220>
   <223> General Hairpin Ribozyme Site
<220>
   <223> General Hairpin Ribozyme Site
<220>
   <221> modified_base
   <222> (1)
   <223> Where n is a, c, g or u
<220>
   <221> modified_base
   <222> (2)
   <223> Where n is a, c, g or u
<220>
   <221> modified base
   <222> (3)
   <223> Where n is a, c, g or u
<220>
   <221> modified base
   <222> (5)
   <223> Where n is a, c, g or u
<220>
   <221> modified_base
   <222> (9)
   <223> Where n is a, c, g or u
<220>
   <221> modified_base
   <222> (10)
   <223> Where n is a, c, g or u
<220>
   <221> modified_base
   <222> (11)
   <223> Where n is a, c, g or u
<220>
   <221> modified_base
   <222> (12)
   <223> Where n is a, c, g or u
<220>
   <221> modified_base
   <222> (13)
   <223> Where n is a, c, g or u
<220>
   <221> modified_base
   <222> (14)
   <223> Where n is a, c, g or u
<220>
   <221> modified_base
   <222> (15)
   <223> Where n is a, c, g or u
<220>
   <221> modified_base
   <222> (16)
   <223> Where n is a, c, g or u
<220>
   <221> modified_base
   <222> (17)
   <223> Where n is a, c, g or u
<220>
   <221> modified_base
   <222> (18)
   <223> Where n is a, c, g or u
<400> 270
   nnnbngucnn nnnnnnnn 18
<210> 271
   <212> DNA
   <213> Homo sapiens
<220>
   <223> Hairpin ribozyme recognition site for PCNA
<400> 271
<210> 272
   <211> 16
   <212> DNA
   <213> Homo sapiens
<220>
   <223> Hairpin ribozyme recognition site for PCNA
<400> 272
   tcgttgtctt tctagg 16
<210> 273
   <211> 16
   <212> DNA
   <213> Homo sapiens
<220>
   <223> Hairpin ribozyme recognition site for PCNA
<400> 273
   gcctggtcca gggctc 16
<210> 274
   <211> 16
   <212> DNA
   <213> Homo sapiens
<220>
   <223> Hairpin ribozyme recognition site for PCNA
<400> 274
   gactcgtccc acgtct 16
<210> 275
   <211> 16
   <212> DNA
   <213> Homo sapiens
<220>
   <223> Hairpin ribozyme recognition site for PCNA
<400> 275
   ctgcggtctg agggct 16
<210> 276
   <211> 16
   <212> DNA
   <213> Homo sapiens
<220>
   <223> Hairpin ribozyme recognition site for PCNA
<400> 276
   aaattgtcac agacaa 16
<210> 277
   <211> 16
   <212> DNA
   <213> Homo sapiens
<220>
   <223> Hairpin ribozyme recognition site for PCNA
<400> 277
   tttctgtcac caaatt 16
<210> 278
   <211> 16
   <212> DNA
   <213> Homo sapiens
<220>
   <223> Hairpin ribozyme recognition site for PCNA
<400> 278
   atctggtcta gttaac 16
<210> 279
   <211> 16
   <212> DNA
   <213> Homo sapiens
<220>
   <223> Hairpin ribozyme recognition site for PCNA
<400> 279
   tttttgtctc ttagaa 16
<210> 280
   <211> 16
   <212> DNA
   <213> Homo sapiens
<220>
   <223> Hairpin ribozyme recognition site for PCNA
<400> 280
   aaagggtctt gactct                                         16
<210> 281
   <211> 39
   <212> DNA
   <213> Homo sapien
<220>
   <223> General structure of chimeric DNA/RNA ribozyme
<220>
   <221> modified_base
   <222> (1..8)
   <223> Where n is a, c, g or t
<220>
   <221> modified_base
   <222> (33..39)
   <223> Where n is a, c, g or t
<400> 281
   nnnnnnnncu gaugagcaat tttttgcgaa aannnnnnn                39
<210> 282
   <211> 16
   <212> DNA
   <213> Homo sapiens
<220>
   <223> General target RNA of chimeric DNA/RNA ribozyme
<220>

## Claims

1. Use of a chimeric DNA/RNA hammerhead ribozyme in the manufacture of a medicament for treatment of a proliferative disease of the eye, wherein the ribozyme is directed to the sequence of any of SEQ ID NOS: 3855-4115, 4143-4152, wherein these sequences are within the PCNA gene.

2. Use according to claim 1, wherein the medicament inhibits retinal detachment.

3. Use according to claim 1 or claim 2, wherein the ribozyme is directed to SEQ ID NO: 4145.

4. Use according to any one of claims 1 to 3 wherein the proliferative disease of the eye is proliferative vitreoretinopathy (PVR).

5. Use according to any one of claims 1 to 3 wherein the proliferative disease of the eye is proliferative diabetic retinopathy (PDR).

## Patentansprüche

1. Verwendung eines chimären DNA/RNA-Hammerkopf-Ribozyms bei der Herstellung eines Medikaments zur Behandlung einer proliferativen Erkrankung des Auges, worin das Ribozym auf die Sequenz nach einer der Seq.-ID Nr. 1-261, 271-280 gerichtet ist, worin diese Sequenzen innerhalb des PCNA-Gens liegen.

2. Verwendung nach Anspruch 1, worin das Medikament Netzhautablösung hemmt.

3. Verwendung nach Anspruch 1 oder Anspruch 2, worin das Ribozym auf Seq.-ID Nr. 273 gerichtet ist.

4. Verwendung nach einem der Ansprüche 1 bis 3, worin die proliferative Erkrankung des Auges proliferative Vitreoretinopathie (PVR) ist.

5. Verwendung nach einem der Ansprüche 1 bis 3, worin die proliferative Erkrankung des Auges proliferative diabetische Retinopathie (PDR) ist.

## Revendications

1. Utilisation d'une ribozyme tête-de-marteau d'ADN/ARN chimérique dans la fabrication d'un médicament pour le traitement d'une maladie proliférative de l'oeil, où la ribozyme est dirigée vers la séquence de l'un quelconque parmi SEQ ID NOS: 1-261, 271-280, ces séquences étant à l'intérieur du gène PCNA.

2. Utilisation selon la revendication 1, dans laquelle le médicament empêche un détachement de la rétine.

3. Utilisation selon la revendication 1 ou la revendication 2, dans laquelle la ribozyme est dirigée vers SEQ ID NO: 273;.

4. Utilisation selon l'une quelconque des revendications 1 à 3 dans laquelle la maladie proliférative de l'oeil est la vitréorétinopathie proliférative (VRP).

5. Utilisation selon l'une quelconque des revendications 1 à 3, dans laquelle la maladie proliférative de l'oeil est le rétinopathie diabétique proliférative (RDP).
